# EUROPÄISCHE PATENTSCHRIFT

(11) **EP 2 855 075 B1**
(45) Veröffentlichungstag und Bekanntmachung des Hinweises auf die Patenterteilung: **23.11.2016**
(21) Anmeldenummer: 13729252.0
(22) Anmeldetag: 29.05.2013
(51) Int. Cl.: A61F 2/01, A61F 2/91, A61B 17/221, B23K 26/364, B23K 26/0622

(54) **VERFAHREN ZUM HERSTELLEN EINES KÖRPERIMPLANTATS**
METHOD FOR PRODUCING A BODY IMPLANT
PROCÉDÉ DE FABRICATION D'UN IMPLANT CORPOREL

(30) Priorität: 30.05.2012 DE 102012010687
(43) Veröffentlichungstag der Anmeldung: 08.04.2015
(73) Patentinhaber: Admedes Schuessler GmbH, 75179 Pforzheim (DE)
(72) Erfinder: FETH, Nils-Agne, 76337 Waldbronn (DE); LANGE, Alexander, 76135 Karlsruhe (DE); STEINER, Ralf, 75173 Pforzheim (DE)
(74) Vertreter: Müller-Boré & Partner Patentanwälte PartG mbB
(86) Internationale Anmeldenummer: PCT/EP2013/001592
(87) Internationale Veröffentlichungsnummer: WO 2013/178361

(56) Entgegenhaltungen:
- DE-A1- 19 722 429
- DE-A1- 19 745 294
- US-A1- 2002 065 553
- US-A1- 2005 182 390
- US-A1- 2010 102 046
- US-A1- 2011 070 358
- US-A1- 2011 245 907

## Beschreibung

Die vorliegende Erfindung bezieht sich auf ein Verfahren zum Herstellen eines Körperimplantats, eine Baugruppe aus einem Führungsdraht und einem Körperimplantat sowie auf ein medizinisches Instrument.

Herkömmlich werden Körperimplantate sowie medizinische Instrumente, wie beispielsweise Stents, Körbchen, Filter oder Catching Devices, aus einem hohlen Rundmaterial, wie beispielsweise einem Rohr, hergestellt. Alternativ oder zusätzlich können diese Vorrichtungen durch Flechten von Drähten hergestellt werden. Dabei ist ein Fertigungsaufwand jedoch hoch und eine Crimpfähigkeit, das heißt eine Fähigkeit einer Durchmesserverringerung zum Einführen in den menschlichen Körper, ist bei derart hergestellten Vorrichtungen begrenzt. Es besteht somit ein Bedarf für ein Verfahren und durch dieses Verfahren hergestellte Vorrichtungen, die eine höhere Crimpfähigkeit aufweisen und einfacher herstellbar sind.

US2002/0065553 A1 offenbart ein Verfahren zur Herstellung einer medizinischen Vorrichtung mit einem beschichteten Abschnitt durch Laserabtrag. Dabei wird ein Ultrakurzpulslaser mit einer Pulsdauer von weniger als 10 Pikosekunden verwendet. Ferner offenbart DE 197 45 294 A1 ein Verfahren zur Herstellung feinstrukturierter, medizintechnischer Implantate mit einem gepulsten Laserstrahl einer Pulsdauer in der Größenordnung von Femtosekunden. Weiterhin beschreibt US 2010/0102046 A1 eine Laserbearbeitungstechnik zur Herstellung medizinischer Vorrichtungen mit lokaler Kühlung.

DE 197 22 429 A1 offenbart eine Vorrichtung zum Einfangen und/oder Zerkleinern von Gegenständen in Hohlorganen, bei der Fangstränge einstückig miteinander ausgebildet sind.

Es ist Aufgabe der Erfindung besteht in der Schaffung eines flexibleren Verfahren zum Herstellen eines Körperimplantats, mit dem Strukturen des Körperimplantants mit einem hohen Freiheitsgrad hergestellt werden können.

Diese Aufgabe wird durch die Merkmale der unabhängigen Ansprüche gelöst. Vorteilhafte Weiterbildungen der Erfindung sind in den abhängigen Ansprüchen definiert.

Gemäß einem Gesichtspunkt betrifft die Erfindung ein Verfahren zum Herstellen eines Körperimplantats mit den Schritten:
Bereitstellen eines Drahts;
Erzeugen von Schnitten bzw. Einkerbungen bzw. Strukturen im Querschnitt des Drahts mittels eines selektiven Abtragungsprozesses, um eine vorgegebene Gestalt des Körperimplantats zu erzeugen,
wobei die Schnitte in radialer Richtung bis etwa die Mitte des Drahts erzeugt werden.

Vorzugsweise wird der selektive Abtragungsprozeß zumindest teilweise mittels eines Ultrakurzpulslasers durchgeführt.

Die Ultrakurzpuls-Lasertechnologie (UKP) ermöglicht vorteilhafterweise die Mikrobearbeitung von Materialien, wie beispielsweise Drähten ohne die Notwendigkeit, das entfernte Material, wie beispielsweise wiedererstarrte Schmelze beim konventionellen Laser-Schmelzschneiden auszutreiben. Es findet also bevorzugt ein kalter Abtragungsprozeß, ein sogenannter Ablationsprozeß statt. Im Gegensatz zum konventionellen Laser-Schmelzschneiden, bei dem ausschließlich durchgehende Schnitte möglich sind, ergibt sich die Möglichkeit, einen Draht zu strukturieren und damit die mechanischen und/oder elektrischen bzw. elektronischen Eigenschaften zu verändern. Beispielsweise erlaubt dieser Prozeß weiterhin vorteilhafterweise sehr feine Drähte symmetrisch und asymmetrisch durch Schnitte aufzuschlitzen. Durch Erzeugen von radialen Schnitten in dem Draht können somit Drahtsegmente erzeugt und gegebenenfalls von dem Draht gelöst werden und anschließend geformt und/oder aufgeweitet werden, um beispielsweise ein Körbchen, einen Stent oder einen Filter zu erzeugen. Darüber hinaus können die Drahtsegmente gegebenenfalls verflochten werden, um mechanische und elektrische und/oder elektronische Eigenschaften gezielt zu beeinflussen.

Vorzugsweise wird ein geschnittener Abschnitt des Drahts oder ein Drahtsegment aufgeweitet, um ein Körperimplantat, wie beispielsweise ein geschlossenes Körbchen oder ein Filter, Stent oder dergleichen zu erzeugen.

Durch die Schnitte kann beispielsweise auch ein Teil, vorzugsweise der größte Teil, des Querschnitts des Drahts entfernt werden, um dadurch ein Gelenk zu erzeugen.

Unter einem Schnitt soll hier jedoch nicht nur ein durchgehender Schnitt verstanden werden, sondern auch eine Struktur bzw. Strukturierung, die gegebenenfalls als nicht durchgehender Einschnitt oder als Einkerbung einer Drahtoberfläche ausgebildet ist. Hierdurch kann insbesondere eine gezielte Querschnittsschwächung erzeugt werden. Es kann aber durch geeignete Schnitt(e) auch eine Struktur zum Erzeugen eines Gelenks und/oder einer Sollbruchstelle erzeugt werden.

Durch gezielte Schwächung des Querschnitts kann eine mechanische Eigenschaft (z.B. ein Verformungsverhalten) des Körperimplantats beispielsweise bei Beanspruchung auf Biegung verändert werden, um hierdurch ein Einführen in den lebenden Körper zu erleichtern. Darüber hinaus können elektrische bzw. elektronische Eigenschaften gezielt beeinflusst werden. Elektrische bzw. elektronische Eigenschaften des Körperimplantats bzw. medizinischen Instruments können somit gezielt eingestellt werden, um insbesondere Wärme bei Durchströmen mit elektrischem Strom gezielt lokal zu erzeugen, um Gewebe (z.B. eines Tumors oder von diesen versorgenden Gefäße) abzutöten.

Weiter bevorzugt kann das Verfahren des weiteren den Schritt des Verflechtens von Drahtsegmenten und/oder einen Schritts einer Formgebung von Drahtsegmenten aufweisen.

Vorzugsweise wird ein Draht aus einem Material mit Formgedächtniseigenschaften, wie beispielsweise Nitinol, verwendet. Es kann jedoch auch ein Polymer oder ein anderes Metallmaterial für den Draht verwendet werden. Beispiele hierfür sind ein Polymer ohne Formgedächtnis, ein Formgedächtnispolymer, ein bioresorbierbares Polymer und/oder ein nicht bioresorbierbares Polymer, und/oder ein Metall, wie beispielsweise ein reines Metall, eine Legierung und/oder eine Formgedächtnislegierung und/oder eine Keramik.

Vorzugsweise kommen bei dem selektiven Abtragungsprozeß zumindest teilweise ein oder mehrere von folgenden Verfahren zum Einsatz: ein elektrochemischer Abtragungsprozess, ein Erodieren, ein elektrochemisches Polieren, ein nasschemisches Ätzen, ein trockenchemisches Ätzen bzw. reaktives Ionenätzen, ein physikalisches Ionenätzen und/oder eine fokussierte Ionenstrahlbearbeitung.

Somit kann für den selektiven Abtragungsprozess ein elektrochemisches Abtragen, ein Erodieren, eine Maskierung mit anschließendem Ätzen oder eine Ionenstrahlbearbeitung (Focused Ion Beam) angewandt werden. Vorzugsweise wird jedoch eine Ultrakurzpuls-Lasertechnologie (UKP) angewandt.

Um aus Drähten medizinische Implantate und Werkzeuge herzustellen, kann ein elektrochemischer Abtragungsprozess angewandt werden, bei dem sich ein Werkstück (d.h. Draht) als Anode und ein Werkzeug als Kathode in einem Elektrolyt befinden. Beim Anlegen einer Spannung fließt ein Strom zwischen Werkstück und Werkzeug. Als Folge des fließenden Stroms werden Metallionen aus dem Werkstück herausgelöst, wodurch das Werkstück mit hoher Auflösung bearbeitet werden kann.

Es kann auch ein Erodieren durchgeführt werden, bei dem eine Spannung zwischen Werkstück (d.h. Draht) und Werkzeug angelegt wird. Das Werkzeug kann auch ein Draht sein. Sowohl Werkstück als auch Werkzeug befinden sich in einem flüssigen Dielektrikum. Beim Anlegen einer elektrischen Spannung springt ein Funken zwischen Werkzeug und Werkstück über, der das Werkstück erwärmt, so dass Material schmilzt und verdampft. Hierdurch findet ein Materialabtrag statt. Dieser Mechanismus findet sich beim Draht- und beim Senkerodieren wieder.

Es besteht die Möglichkeit, den Draht mit einer Maske zu versehen, beispielsweise aus Photolack. Die Maske schirmt den Draht von äußeren Einflüssen ab. Nun kann ein flächiger Abtragungsmechanismus genutzt werden, um Material gezielt an den nicht maskierten Bereichen des Drahts abzutragen und den Draht somit zu bearbeiten. Nach dem Abtragen wird die Maske entfernt. Als flächige Abtragungsmechanismen kommen elektrochemisches Polieren, nasschemisches Ätzen, trockenchemisches Ätzen bzw. reaktives Ionenätzen und physikalisches Ionenätzen in Frage.

Bei der fokussierten Ionenstrahlbearbeitung (Focused Ion Beam)_wird das Werkstück (d.h. Draht) mit fokussierten, hochenergetischen (keV) Ionen (bspw. geladene Ar⁺-Ionen) beschossen. Die Ionen tragen Material in einem eng begrenzten lokalen Bereich von der Oberfläche ab. Die Auflösung beträgt wenige Nanometer.

Die Ultrakurzpuls-Lasertechnologie (UKP) ermöglicht die Mikrobearbeitung von Materialien, wie beispielsweise Drähten ohne die Notwendigkeit, das entfernte Material, wie beispielsweise wiedererstarrte Schmelze beim konventionellen Laser-Schmelzschneiden auszutreiben. Es findet also ein kalter Abtragungsprozeß, ein sogenannter Ablationsprozeß statt. Im Gegensatz zum konventionellen Laser-Schmelzschneiden, bei dem ausschließlich durchgehende Schnitte möglich sind, ergibt sich die Möglichkeit, einen Draht einerseits zu strukturieren und damit die mechanischen und/oder elektrischen bzw. elektronischen Eigenschaften zu verändern. Andererseits erlaubt dieser Prozeß sehr feine Drähte symmetrisch und asymmetrisch durch Schnitte aufzuschlitzen. Durch Erzeugen von radialen Schnitten in dem Draht können somit Drahtsegmente von dem Draht gelöst werden und anschließend geformt und/oder aufgeweitet werden, um beispielsweise ein Körbchen, einen Stent oder einen Filter zu erzeugen. Darüber hinaus können die Drahtsegmente verflochten werden, um mechanische und elektrische bzw. elektronische Eigenschaften gezielt zu beeinflussen.

Darüber hinaus können die Schnitte bzw. Einkerbungen bzw. Strukturen so erzeugt werden, daß ein einstückig angeformtes Gelenk aus dem Draht erzeugt wird, indem ein großer Teil des Querschnitts entfernt wird. Somit ergeben sich vielzählige Gestaltungsmöglichkeiten, indem an einem Draht entsprechende Schnitte mit der Ultrakurzpuls-Lasertechnologie erzeugt werden.

Vorzugsweise werden durch Ausbildung von ein oder mehreren Schnitten mittels des selektiven Abtragungsprozesses ein oder mehrere Strukturen auf bzw. in dem Draht ausgebildet. Dabei umfasst die Struktur vorzugsweise zumindest eine der folgenden Ausbildungen: ein Gelenk, eine Einkerbung, einen Einschnitt, eine Sollbruchstelle und/oder eine Querschnittsschwächung.

Gemäß einem weiteren Gesichtspunkt betrifft die Erfindung eine Baugruppe aus einem Führungsdraht und einem Körperimplantat, die mit einem derartigen Verfahren hergestellt ist, wobei Führungsdraht und Körperimplantat einstückig bzw. monolithisch ausgebildet sind und eine Sollbruchstelle aufweisen. Dabei kann der Führungsdraht zumindest ein einstückig ausgebildetes Gelenk aufweisen, um eine Flexibilität bzw. Biegsamkeit des Führungsdrahts zu erhöhen.

Vorzugsweise weist die Baugruppe zumindest eines aus einem Stent, einem Körbchen, oder einem Filter auf.

Gemäß einem weiteren Gesichtspunkt wird ein medizinisches Instrument mit einem Führungsdraht zur Verfügung gestellt, das mit diesem Verfahren hergestellt ist. Dabei kann der Führungsdraht zumindest ein einstückig ausgebildetes Gelenk aufweisen.

Diese Fertigungstechnologie hat den Vorteil, daß eine Baugruppe aus einem Führungsdraht und einem Körperimplantat bzw. einem medizinischen Instrument aus einem Stück herstellbar ist, so daß ein Verbinden beispielsweise durch Mikroschweißen eines Führungsdrahts mit einem Körperimplantat nicht mehr durchgeführt werden muß. Somit wird eine Fehleranfälligkeit einer entsprechenden Baugruppe aus einem Führungsdraht und einem medizinischen Instrument wesentlich verringert.

Diese einstückig ausgebildete Baugruppe kann eine Sollbruchstelle aufweisen, so daß das Körperimplantat bzw. medizinische Instrument nach dem Plazieren in dem menschlichen Körper an der Sollbruchstelle von dem Führungsdraht getrennt wird, um den Führungsdraht zu entfernen. Des weiteren ist der Herstellungsprozess stark vereinfacht. Darüber hinaus kann der Führungsdraht ein einstückig ausgebildetes Gelenk aufweisen, um eine höhere Flexibilität aufzuweisen.

Diese Technologie hat den Vorteil von geringeren Kosten für das Rohmaterial, ist mechanisch stabiler und hat eine maximale Crimpfähigkeit, weil die aufgeweiteten Drahtsegmente bis zur ursprünglichen Lage des nicht geschnittenen Drahts verformt bzw. gecrimpt werden können, um den Durchmesser zum Einführen in den menschlichen Körper auf einen minimalen Durchmesser zu verringern. Darüber hinaus kann die Vorrichtung mit einer minimalen Anzahl von Fertigungsschritten erzeugt werden.

Die Erfindung wird nun anhand von Ausführungsbeispielen unter Bezugnahme auf die Zeichnungen näher erläutert.
Fig. 1 zeigt einen kreisrunden Draht, der durch einen Laserstrahl in radialer Richtung in etwa bis zu dessen Mitte geschnitten wird.
Fig. 2 zeigt das Erzeugen von insgesamt fünf Schnitten in einer radialen Richtung bis zur Mitte des Drahts.
Fig. 3 zeigt ein Körbchen oder Filterdesign, das durch die Schnitte erzeugt wird, indem die abgeschnittenen Drahtsegmente anschließend aufgeweitet werden.
Fig. 4 zeigt einen Führungsdraht, der durch Erzeugen entsprechender Schnitte eine hohe Biegsamkeit bzw. Flexibilität aufweist.
Fig. 5 zeigt einen Stent, der einstückig mit dem Führungsdraht hergestellt ist und eine Sollbruchstelle aufweist.
Fig. 6 zeigt einen steuerbaren Führungsdraht.
Fig. 7 zeigt eine Detailansicht des Führungsdrahts von Fig. 6 mit einem entsprechenden Gelenk zum Abknicken des Führungsdrahts.
Fig. 8a zeigt ein Drahtsegment, das als Kreisausschnitt geformt ist, und Fig. 8b zeigt ein Drahtsegment, das als Kreisabschnitt geformt ist.

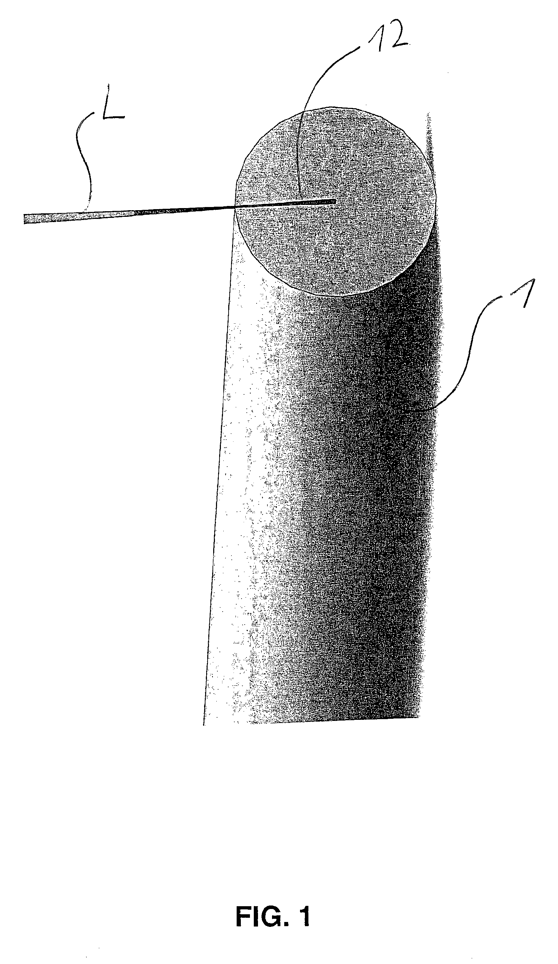

Wie in Fig. 1 gezeigt ist, wird ein kreisrunder Draht 1 durch einen Laserstrahl L mittels der Ultrakurzpuls-Lasertechnologie bis etwa zu dessen Mitte in radialer Richtung eingeschnitten, um einen Schnitt 12 zu erzeugen. Obwohl es vorteilhaft ist, kreisrunde Drähte für diese Technologie anzuwenden, ist die Erfindung nicht darauf beschränkt. Es können auch Drähte mit elliptischem Querschnitt, Dreikantdrähte, Vierkantdrähte, Fünfkantdrähte oder dergleichen verwendet werden.

Vorteilhafterweise wird dabei ein Ultrakurzpuls-Laser mit einer Wellenlänge von etwa 200 bis etwa 2000 nm eingesetzt. Die Impulsdauer sollte etwa 10 fs bis etwa 10 ps betragen.

Der selektive Abtragungsprozess kann jedoch alternativ oder zusätzlich eines aus einem elektrochemischen Abtragen, einem Erodieren, einer Maskierung mit anschließendem Ätzen oder einer Ionenstrahlbearbeitung (Focused Ion Beam) aufweisen.

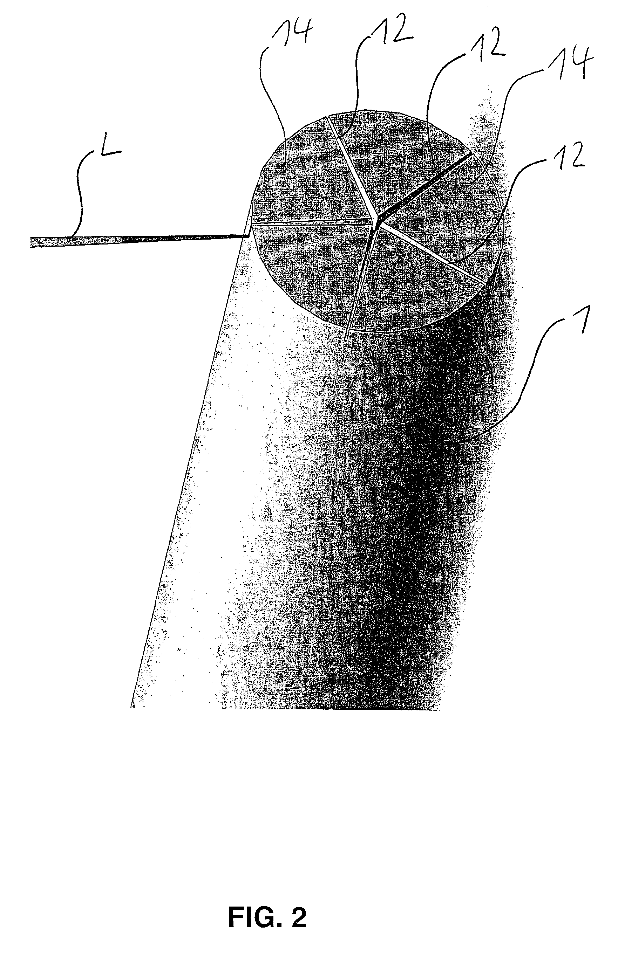

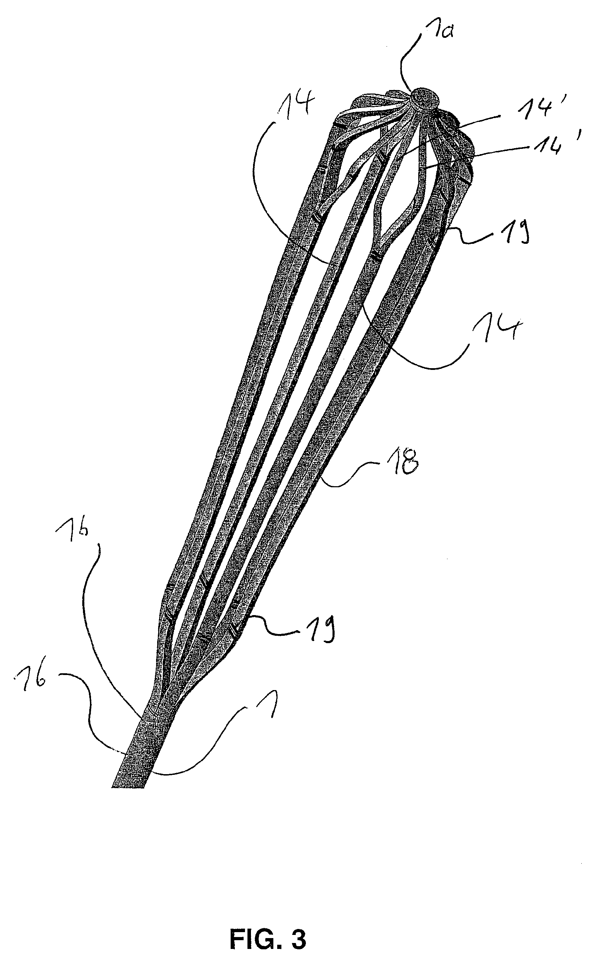

Wie des weiteren in Fig. 2 gezeigt ist, können beispielsweise fünf radiale Schnitte bis zur Mitte des Drahts 1 erzeugt werden, um Drahtsegmente oder geschnittene Abschnitte 14 zu erzeugen. Diese geschnittenen Abschnitte oder Drahtsegmente 14 können anschließend durch Verformen aufgeweitet werden, um beispielsweise ein Körbchen oder Filterdesign zu erzeugen, wie es beispielsweise in Fig. 3 gezeigt ist. Dabei gehen die Schnitte nicht bis zu einem axialen Ende 1 a, 1 b des Drahts 1, so daß die einzelnen Drahtsegmente 14 an ihren Enden in Längsrichtung noch miteinander verbunden sind.

Auf diese Weise wird aus einem Draht 1 ein Körbchen bzw. Filter erzeugt, das Drahtsegmente 14 an den Enden 1a, 1 b einstückig oder integral verbunden aufweist.

In anderen Worten wird der Draht 1 wie ein Kuchen in einzelne Segmente (Drahtsegmente 14) aufgetrennt, wobei axiale Enden 1 a, 1 b des Drahts nicht getrennt werden. Die aufgetrennten Drahtsegmente 14 werden aufgeweitet, so dass ein Körper mit beabstandeten Drahtsegmenten 14 in der axialen Mitte und einstückig mit den Drahtsegmenten 14 verbundenen axialen Enden 1 a, 1 b geformt wird.

Um das Aufweiten zu erleichtern bzw. ein Verformungsverhalten des Körperimplantats zu beeinflussen, können Einschnitte 19 oder Einkerbungen zur gezielten Querschnittsschwächung an dem Draht 1 erzeugt werden, wie in Fig. 3 gezeigt ist. Insbesondere sind die Einschnitte 19 bzw. Einkerbungen an ein oder mehreren Bereichen vorgesehen, an denen ein Steg bzw. Drahtsegment 14 in zwei oder mehrere Unterteilungen 14' unterteilt wird und/oder an denen zwei oder mehr Drahtsegmente 14 vereint werden (siehe oberen Bereich von Fig. 3 nahe dem axialen Ende 1 a). Ferner können ein oder mehrere Einschnitte 19 bzw. Einkerbungen in oder bei ein oder mehreren Bereichen des Stegs bzw. Drahtsegments 14 vorgesehen sein (siehe unteren Bereich von Fig. 3 nahe dem axialen Ende 1), in oder bei denen das jeweilige Drahtsegment bzw. Steg 14 durchgehend (d.h. nicht unterteilt) ausgebildet sind, wobei bevorzugt bei oder in Nähe der Einschnitt(e) 19 das jeweilige Drahtsegment bzw. Steg 14 gebogen oder nicht-linear ausgestaltet ist.

Durch die ein oder mehreren Schnitte, insbesondere durch die Querschnittsschwächung, können die elektrischen oder elektronischen Eigenschaften des entsprechenden Bereichs des Körperimplantats oder des medizinischen Instruments, insbesondere der elektrische Widerstand, so verändert bzw. eingestellt werden, dass beim Anlegen eines elektrischen Stroms lokal Wärme zum Abtöten von Körpergewebe erzeugt wird.

Es versteht sich, dass die Erfindung auch mit drei, vier, sechs oder sieben Schnitten 12 etc. ausgeführt werden kann. Dabei müssen die Schnitte 12 nicht in gleichmässigen Winkelabständen durchgeführt werden, sondern es können unterschiedlich große "Kuchenstücke" bzw. Drahtsegmente 14 erzeugt werden.

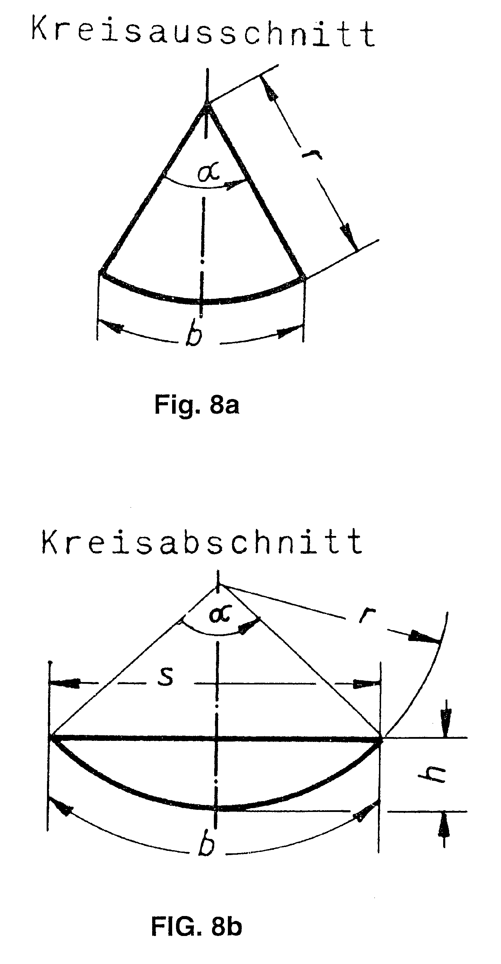

Darüber hinaus müssen die Drahtsegmente 14 nicht als Kreisausschnitte mit der Länge des Radius r des Drahts 1 als Seiten des Kreisausschnitts erzeugt werden, d.h. durch Schnitte 12, die im Mittelpunkt des Drahtquerschnitts enden, wie in Fig. 8a gezeigt ist, sondern es können beliebige Formen, wie beispielsweise ein Kreisabschnitt von dem Drahtquerschnitt getrennt werden, wie beispielsweise in Fig. 8b gezeigt ist.

Ein Kreisabschnitt, wie in Fig. 8b gezeigt ist, wird dadurch von dem Drahtquerschnitt herausgetrennt, dass ein Schnitt 12 versetzt zu dem Mittelpunkt des Drahtquerschnitts entlang der Sehnenlänge s erzeugt wird. Dabei ist eine Höhe h des Schnitts 12 geringer als der Radius r des Drahts.

Indem auf diese Weise Drahtsegmente 14 mit unterschiedlichen Querschnitten von dem Drahtquerschnitt getrennt und anschließend aufgeweitet werden, kann beispielsweise ein Stent erzeugt werden, der unterschiedliche Stützkräfte entlang seines Umfangs aufweist.

Diese Drahtsegmente 14 können einer weiteren Formgebung durch Umformen oder weitere Laserbearbeitung entsprechend den gewünschten mechanischen und/oder elektrischen Eigenschaften verformt oder bearbeitet werden. Darüber hinaus können die Drahtsegmente 14 verflochten werden, beispielsweise.durch Klöppeln der Drahtsegmente mit den Bindungsarten 1/2, 1/1 oder 2/2. Die Drahtsegmente 14 können daher entsprechende Stege 14 eines Stents 18 ausbilden.

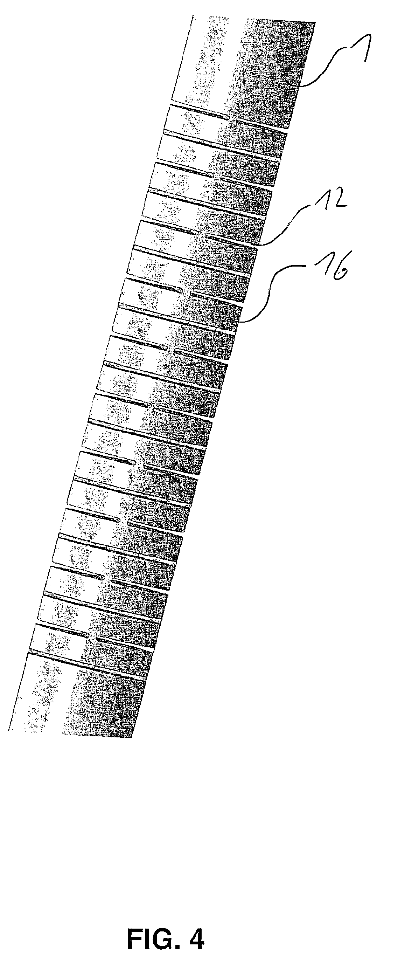

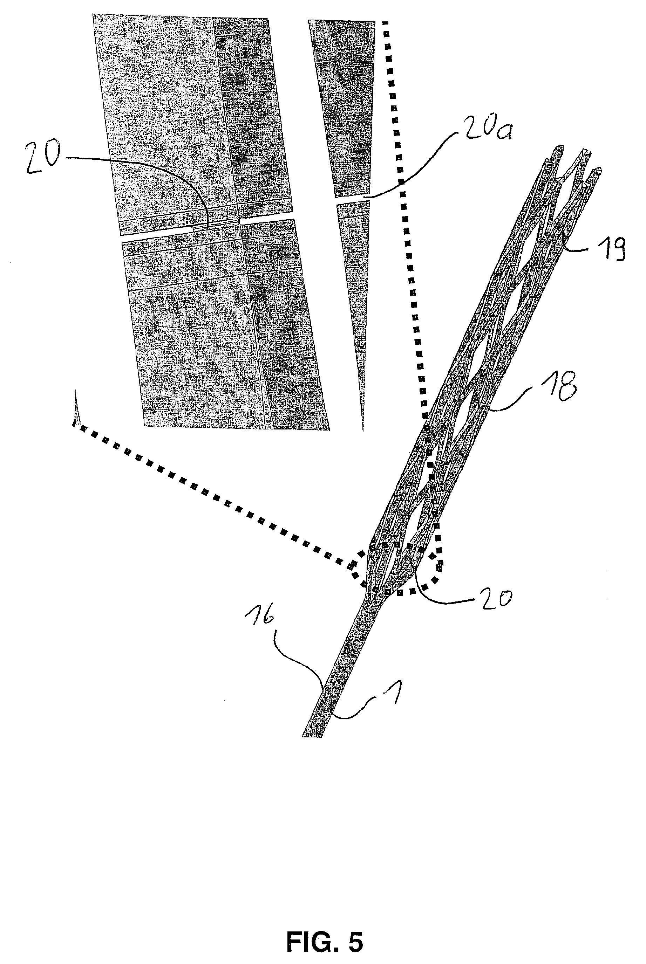

Das somit erzeugte Körbchen oder Filterdesign bzw. Stent 18 kann anschließend vom Draht 1 getrennt werden. Es kann jedoch auch an einem längeren Stück Draht 1 verbleiben, so daß das nicht geschnittene Ende des Drahts 1 als Führungsdraht 16 verwendet wird, wie beispielsweise in Figs. 4 und 5 gezeigt ist.

Zwischen dem Führungsdraht 16 und dem erzeugten Körperimplantat 18 kann sich dabei eine Sollbruchstelle 20 befinden, so daß die Baugruppe aus dem Führungsdraht 16 und dem Körperimplantat 18, die auf diese Weise einstückig erzeugt wird, nach dem Einführen und Plazieren des Körperimplantats 18 an der Sollbruchstelle 20 getrennt werden kann, um den Führungsdraht 16 nach dem Plazieren des Körperimplantats 18 zu entfernen. Der Führungsdraht 16 kann dabei wie in Fig. 4 gezeigt ist, entsprechende Schnitte 12 bzw. Querschnittsschwächungen bzw. Strukturen aufweisen, um eine hohe Biegsamkeit bzw. Flexibilität zu erzeugen. Die Sollbruchstelle 20 hat eine entsprechende Querschnittsschwächung 20a, um ein Abbrechen oder Ablösen an der Sollbruchstelle 20 zu erleichtern.

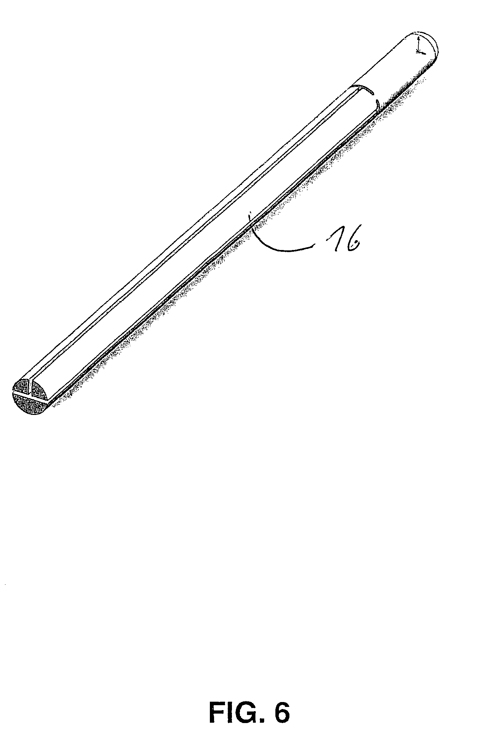

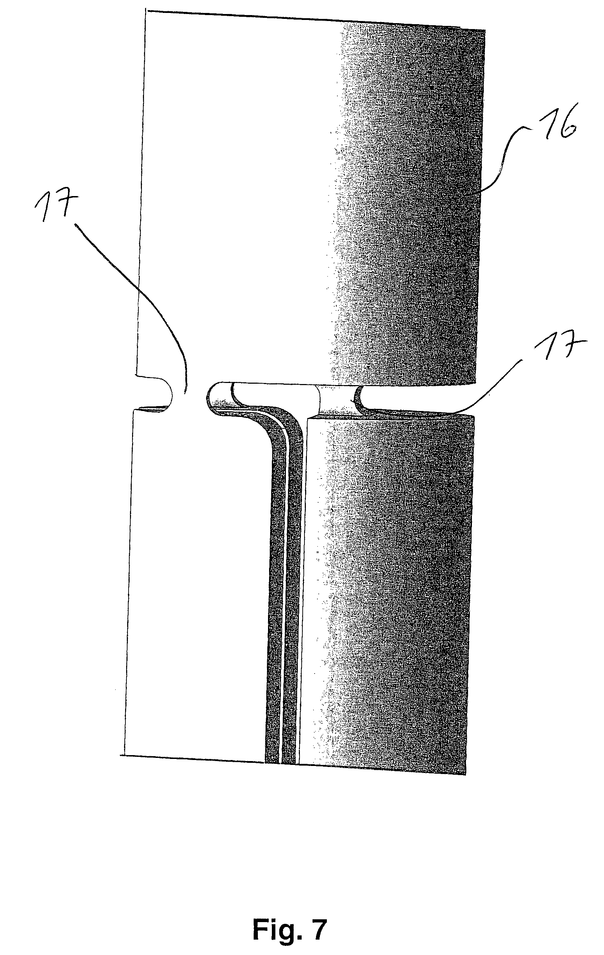

Wie des weiteren in den Figuren 6 und 7 gezeigt ist, kann durch einen Schnitt, bei dem fast der gesamte Querschnitt des Drahts bzw. Führungsdrahts 16 entfernt wird, ein entsprechendes Gelenk 17 erzeugt werden, um das ein Ende des Führungsdrahts 16 gegenüber einem anderen Abschnitt gebogen werden kann.

Es ergeben sich somit vielfältige Designmöglichkeiten, indem ein Draht 1 durch den selektiven Abtragungsprozess, wie beispielsweise die UKP-Lasertechnologie, entsprechend geschnitten wird und die geschnittenen Drahtsegmente 14 eventuell aufgeweitet und/oder weiter bearbeitet werden. Auf diese Weise kann ein Körperimplantat 18, wie beispielsweise ein Stent, ein Körbchen oder ein Filter, einstückig auf einfache Weise hergestellt werden. Darüber hinaus kann dieses Körperimplantat 18 bis zum ursprünglichen Drahtdurchmesser gecrimpt werden, um eine maximale Durchmesserverringerung zu erzielen.

Vorzugsweise wird ein Draht 1 aus einem Material mit Formgedächtniseigenschaften wie beispielsweise Nitinol verwendet. Es können jedoch auch andere Metalle oder auch Nichtmetalle, wie beispielsweise Polymere verwendet werden. Beispiele hierfür sind ein Polymer ohne Formgedächtnis, ein Formgedächtnispolymer, ein bioresorbierbares Polymer und/oder ein nicht bioresorbierbares Polymer, und/oder ein Metall, wie beispielsweise ein reines Metall, eine Legierung und/oder eine Formgedächtnislegierung, und/oder eine Keramik.

Die Erfindung ist nicht auf das Erzeugen von radialen Schnitten 12 beschränkt, die Schnitte können auch in anderen Richtungen erzeugt werden. Darüber hinaus müssen die Schnitte 12 nicht bis zur Mitte des Drahts 1 gehen, sondern können, je nach Anwendungsfall, weniger tief oder tiefer erzeugt werden. Hierdurch können Strukturen oder Einkerbungen an dem Querschnitt des Drahts 1 erzeugt werden.

### Bezugszeichenliste

- 1: Draht
- 12: Schnitt
- 14: Drahtsegment (Steg)
- 14': Unterteilung
- 16: Führungsdraht
- 17: Gelenk (Struktur)
- 18: Körperimplantat (Stent)
- 19: Einkerbung bzw. Einschnitt (Struktur)
- 20: Sollbruchstelle (Struktur)
- 20a: Querschnitttsschwächung (Struktur)
- L: Laser

## Patentansprüche

1. Verfahren zum Herstellen eines Körperimplantats (18) oder medizinischen Instruments mit den Schritten:
Bereitstellen eines Drahts (1);
Erzeugen von vorgegebenen Schnitten (12) an dem Draht (1) mittels eines selektiven Abtragungsprozesses, um eine vorgegebene Gestalt des Körperimplantats (18) zu erzeugen,
**dadurch gekennzeichnet, dass**
die Schnitte (12) in radialer Richtung bis etwa die Mitte des Drahts (1) erzeugt werden.

2. Verfahren nach Anspruch 1, wobei der selektive Abtragungsprozeß zumindest teilweise mittels eines Ultrakurzpulslasers (L) durchgeführt wird.

3. Verfahren nach einem der vorangehenden Ansprüche, wobei ein geschnittener Abschnitt (14) des Drahts (1) oder ein Drahtsegment aufgeweitet wird, um ein geschlossenes Körbchen oder ein Filter zu erzeugen.

4. Verfahren nach einem der vorherigen Ansprüche, wobei durch die Schnitte (12) ein Teil des Querschnitts des Drahts (1) entfernt wird, um ein Gelenk (17) zu erzeugen.

5. Verfahren nach einem der vorherigen Ansprüche, des weiteren mit dem Schritt des Verflechtens bzw. des Klöppelns von Drahtsegmenten und/oder eines Schritts einer Formgebung von Drahtsegmenten.

6. Verfahren nach einem der vorherigen Ansprüche, wobei ein Draht (1) aus einem Polymer, wie bespielsweise einem Polymer ohne Formgedächtnis, einem Formgedächtnispolymer, einem bioresorbierbaren Polymer oder einem nicht bioresorbierbaren Polymer, und/oder einem Metall, wie beispielsweise einem reinen Metall, einer Legierung und/oder einer Formgedächtnislegierung, und/oder einer Keramik verwendet wird.

7. Verfahren nach einem der vorherigen Ansprüche, wobei bei dem selektiven Abtragungsprozeß zumindest teilweise ein oder mehrere von folgenden Verfahren zum Einsatz kommen: ein elektrochemischer Abtragungsprozess, ein Erodieren, ein elektrochemisches Polieren, ein nasschemisches Ätzen, ein trocken-chemisches Ätzen, ein reaktives Ionenätzen, ein physikalisches Ionenätzen und/oder eine fokussierte Ionenstrahlbearbeitung.

8. Verfahren nach einem der vorherigen Ansprüche, wobei durch Ausbildung von Schnitten mittels des selektiven Abtragungsprozesses ein oder mehrere Strukturen (17; 19; 20; 20a) auf bzw. in dem Draht (1) ausgebildet werden.

9. Verfahren nach Anspruch 8, wobei die ausgebildete Struktur zumindest eine der folgenden Ausbildungen umfasst: ein Gelenk (17), eine Einkerbung (19), einen Einschnitt, eine Sollbruchstelle (20) und/oder eine Querschnittsschwächung (20a).

10. Baugruppe aus einem Führungsdraht (16) und einem Körperimplantat (18) oder medizinischen Instrument, die mit einem Verfahren nach einem der vorherigen Ansprüche hergestellt ist, wobei Führungsdraht (16) und Körperimplantat (18) einstückig ausgebildet sind und eine Sollbruchstelle (20) aufweisen.

11. Baugruppe nach Anspruch 10, wobei der Führungsdraht (1) zumindest ein einstückig ausgebildetes Gelenk (17) aufweist.

12. Baugruppe nach einem der Ansprüche 10 oder 11, wobei das Körperimplantat (18) oder das medizinische Instrument zumindest eines aus einem Stent, einem Körbchen, oder einem Filter aufweist.

13. Medizinisches Instrument mit einem Führungsdraht (16), das mit einem Verfahren nach einem der Ansprüche 1 bis 9 hergestellt ist.

14. Medizinisches Instrument nach Anspruch 13, wobei der Führungsdraht (16) zumindest ein einstückig ausgebildetes Gelenk (17) aufweist.

## Claims

1. Method for the production of a body implant (18) or medical instrument comprising the steps of:
providing a wire (1);
producing specified cuts (12) at the wire (1) by means of a selective removal process in order to create a specified shape of the body implant (18),
**characterized in that**
the cuts (12) are being produced in radial direction until approximately the middle of the wire (1).

2. Method according to claim 1, wherein the selective removal process is carried out at least in part by means of an ultrashort pulse laser (L).

3. Method according to one of the previous claims, wherein a cut section (14) of the wire (1) or a wire segment are expanded in order to produce a closed basket or a filter.

4. Method according to one of the previous claims, wherein a part of the cross section of the wire (1) is removed by the cuts (12) in order to produce a joint or hinge (17).

5. Method according to one of the previous claims, furthermore comprising the step of intertwining or lacing, respectively, and/or a step of a shaping wire segments.

6. Method according to one of the previous claims, wherein a wire (1) is used made of a polymer, such as for instance a polymer without shape memory, a shape memory polymer, a bio-absorbable polymer or a non-bio-absorbable polymer, and/or a metal, such as for instance a pure metal, an alloy and/or a shape memory alloy, and/or a ceramic.

7. Method according to one of the previous claims, wherein in the selective removal process at least partly one or more of the following procedures are used: an electro-chemical removal process, an eroding, an electro-chemical polishing, a wet-chemical etching, a dry etching, a reactive ion etching, a physical ion etching and/or a focused ion beam treatment.

8. Method according to one of the preceding claims, wherein by means of the formation of cuts by means of the selective removal process one or more structures (17; 19; 20; 20a) are formed on or in the wire (1), respectively.

9. Method according to claim 8, wherein the formed structure comprises at least one of the following forms: a joint or hinge (17), a notch (19), an incision, a predetermined breaking point (20) and/or a sectional weakening (20a).

10. Assembly of a guide wire (16) and a body implant (18) or medical instrument, which is produced by a method according to one of the preceding claims, wherein the guide wire (16) and the body implant (18) are integrally formed and have a predetermined breaking point (20).

11. Assembly according to claim 10, wherein the guide wire (1) has at least one integrally formed joint or hinge (17).

12. Assembly according to one of the claims 10 or 11, wherein the body implant (18) or the medical instrument have at least one of a stent, a basket or a filter.

13. Medical instrument with a guide wire (16), which is produced by a method according to one of the claims 1 to 9.

14. Medical instrument according to claim 13, in which the guide wire (16) has at least one integrally formed joint or hinge (17).

## Revendications

1. Procédé de fabrication d'un implant corporel (18) ou instrument médical avec les étapes :
mise à disposition d'un fil (1) ;
génération de coupes prédéfinies (12) sur le fil (1) au moyen d'un processus d'enlèvement sélectif pour générer une configuration prédéfinie de l'implant corporel (18),
**caractérisé en ce que**
les coupes (12) sont générées dans la direction radiale jusqu'à environ la moitié du fil (1).

2. Procédé selon la revendication 1, dans lequel le processus d'enlèvement sélectif est réalisé au moins partiellement au moyen d'un laser à impulsions ultracourtes (L).

3. Procédé selon une des revendications précédentes, dans lequel une section découpée (14) du fil (1) ou un segment de fil est élargi(e) pour générer une petite corbeille fermée ou un filtre.

4. Procédé selon une des revendications précédentes, dans lequel une partie de la section transversale du fil (1) est éliminée par les coupes (12) pour générer une articulation (17).

5. Procédé selon une des revendications précédentes, en outre avec l'étape de l'entrelacement ou du tressage de segments de fil et/ou d'une étape d'un façonnage de segments de fil.

6. Procédé selon une des revendications précédentes, dans lequel un fil (1) en un polymère, comme par exemple un polymère sans mémoire de forme, un polymère à mémoire de forme, un polymère biorésorbable ou un polymère non biorésorbable, et/ou un métal, comme par exemple un métal pur, un alliage et/ou un alliage à mémoire de forme, et/ou une céramique est utilisé.

7. Procédé selon une des revendications précédentes, dans lequel un ou plusieurs des procédés suivants sont au moins partiellement utilisés lors du processus d'enlèvement sélectif: un processus d'enlèvement électrochimique, une érosion, un polissage électrochimique, un décapage chimique humide, un décapage chimique sec, un décapage ionique réactif, un décapage ionique physique et/ou un usinage par faisceau ionique concentré.

8. Procédé selon une des revendications précédentes, dans lequel une ou plusieurs structures (17 ; 19 ; 20 ; 20a) sont réalisées sur ou dans le fil (1) par réalisation de coupes au moyen du processus d'enlèvement sélectif.

9. Procédé selon la revendication 8, dans lequel la structure réalisée comprend au moins une des réalisations suivantes : une articulation (17), une entaille (19), une encoche, un point destiné à la rupture (20) et/ou un affaiblissement de section transversale (20a).

10. Module constitué d'un fil de guidage (16) et d'un implant corporel (18) ou instrument médical qui est fabriqué avec un procédé selon une des revendications précédentes, dans lequel le fil de guidage (16) et l'implant corporel (18) sont réalisés d'une pièce et présentent un point destiné à la rupture (20).

11. Module selon la revendication 10, dans lequel le fil de guidage (1) présente au moins une articulation réalisée d'une pièce (17).

12. Module selon une des revendications 10 ou 11, dans lequel l'implant corporel (18) ou l'instrument médical présente au moins un élément parmi un stent, une petite corbeille ou un filtre.

13. Instrument médical avec un fil de guidage (16) qui est fabriqué avec un procédé selon une des revendications 1 à 9.

14. Instrument médical selon la revendication 13, dans lequel le fil de guidage (16) présente au moins une articulation réalisée d'une pièce (17).
